# EUROPEAN PATENT SPECIFICATION

(11) **EP 1 925 257 B1**
(45) Date of publication and mention of the grant of the patent: **19.08.2009**
(21) Application number: 07021094.3
(22) Date of filing: 29.10.2007
(51) Int. Cl.: A61B 8/00

(54) **Portable ultrasound system**
Tragbares Ultraschallsystem
Système d'ultrasons portable

(30) Priority: 23.11.2006 KR 20060116523
(43) Date of publication of application: 28.05.2008
(73) Proprietor: MEDISON CO., LTD., Kangwon-do 250-870 (KR)
(72) Inventor: Song, Young Seuk, Gangnam-gu, Seoul 135-280 (KR); Kim, Jae Gyoung, Gangnam-gu, Seoul 135-280 (KR)
(74) Representative: Schmid, Wolfgang

(56) References cited:
- WO-A-20/06040697
- US-A1- 2002 173 721
- US-A1- 2003 073 894
- US-B1- 6 468 212

## Description

The present application claims priority from Korean Patent Application No. 10-2006-0116523 filed on November 23, 2006.

### BACKGROUND

### 1. Field

The present invention generally relates to an ultrasound system, and more particularly to a portable ultrasound system.

### 2. Background

An ultrasound system has a wide range of applications and has become one of the important diagnostic devices. Specifically, due to its non-invasive and non-destructive nature, the ultrasound system has been extensively used in the medical profession. Modem high-performance ultrasound systems are commonly used to produce two or three-dimensional images of internal features of a target object.

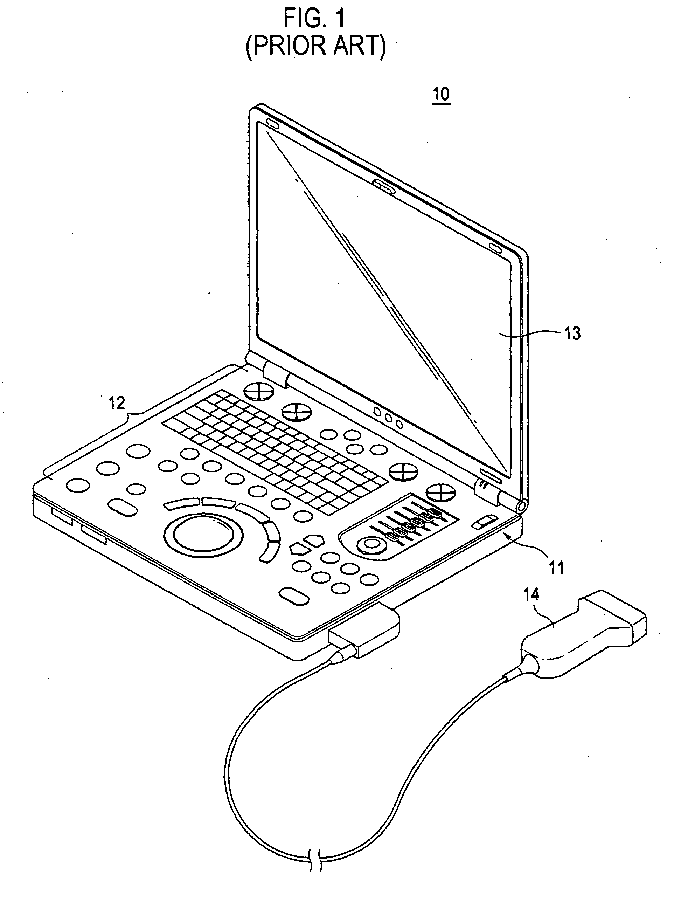

Since conventional ultrasound systems are extremely large and heavy, they must be fixed to a specific location. Further, even the compact versions of such ultrasound systems weigh more than 10 kg and thus cannot be easily moved or carried. In order to resolve this problem, portable ultrasound systems have been developed. A conventional portable ultrasound system is described below with reference to FIG. 1.

FIG. 1 illustrates a conventional portable ultrasound system 10. As shown in FIG. 1, the conventional portable ultrasound system 10 comprises a main unit 11, a control panel 12, a display unit 13 and a probe 14.

The main unit 11 forms the exterior of the above system 10 and receives power for driving the system 10 from an internal battery or an external power supplier. The main unit 11 comprises: a beam former for transmit-focusing ultrasound signals to be transmitted from the probe 14 and receive-focusing ultrasound signals received through the probe 14; a data generator for generating frame data based on the signals from the beam former; a processor for generating two or three-dimensional images of a target object based on the frame data; and a storage unit for storing the data.

The control panel 12 is arranged on the main unit 11 and comprises various input sections for controlling the system 10 to obtain ultrasound images, manipulating menus and performing measurement and annotation functions.

The display unit 13 displays the data and images, which are processed in and provided from the main unit 11.

The probe 14 comprises at least one transducer (not shown). The transducers transmit ultrasound signals to a target object and receive the ultrasound signals reflected from the target object.

The portable ultrasound system 10 may be carried due to its minimized size and weight. However, the conventional portable ultrasound system 10 cannot have a control panel as large as that of a normal-sized ultrasound system on the main unit 11 due to its limited size. This causes problems in that the number of input sections to be included in the control panel 12 must be limited.

A portable ultrasound system according to the preamble of claim 1 is known from WO 2006/040697A1.

### BRIEF DESCRIPTION OF THE DRAWINGS

Arrangements and embodiments may be described in detail with reference to the following drawings in which like reference numerals refer to like elements and wherein:

FIG. 1 illustrates a conventional portable ultrasound system;

FIG. 2 illustrates a portable ultrasound system constructed in accordance with one embodiment of the present invention;

FIG. 3 is a block diagram showing the configuration of a portable ultrasound system constructed in accordance with one embodiment of the present invention; and

FIGS. 4 and 5 illustrate exemplary control panel screens constructed in accordance with one embodiment of the present invention.

### DETAILED DESCRIPTION OF THE PRESENT INVENTION

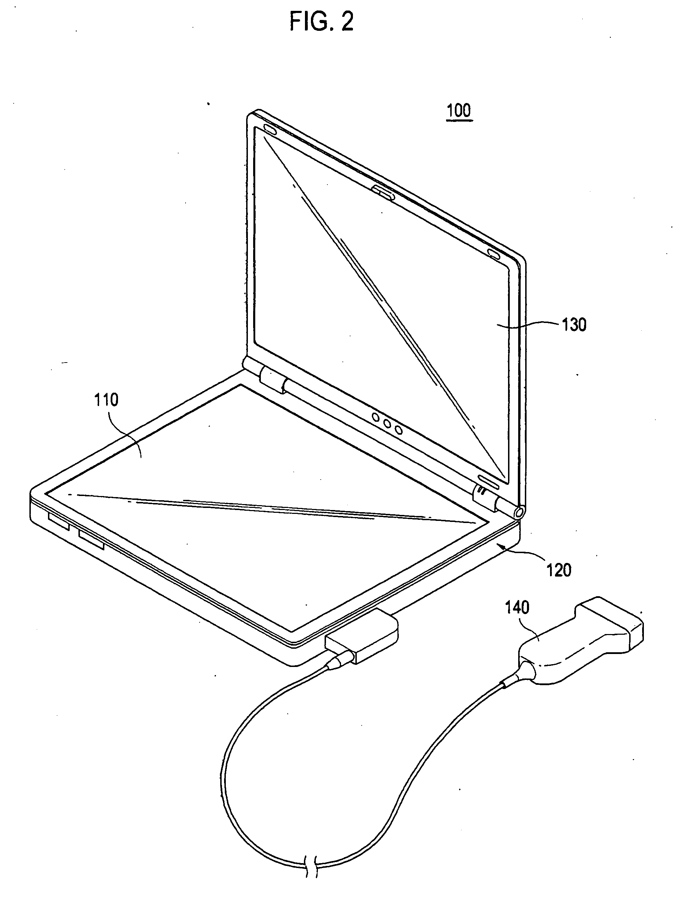

A detailed description may be provided with reference to FIGS. 2-5. One of ordinary skill in the art may realize that the following description is illustrative only and is not in any way limiting. Other embodiments of the present invention may readily suggest themselves to such skilled persons having the benefit of this disclosure.

FIG. 2 illustrates a portable ultrasound system 100 constructed in accordance with one embodiment of the present invention. As shown in FIG. 2, the portable ultrasound system 100 of the present invention comprises a touch panel 110, a main unit 120, a display unit 130 and a probe 140.

The touch panel 110 displays a menu screen including a plurality of input sections to allow a user to manipulate the portable ultrasound system 100. The touch panel 110 generates an output signal from an external input.

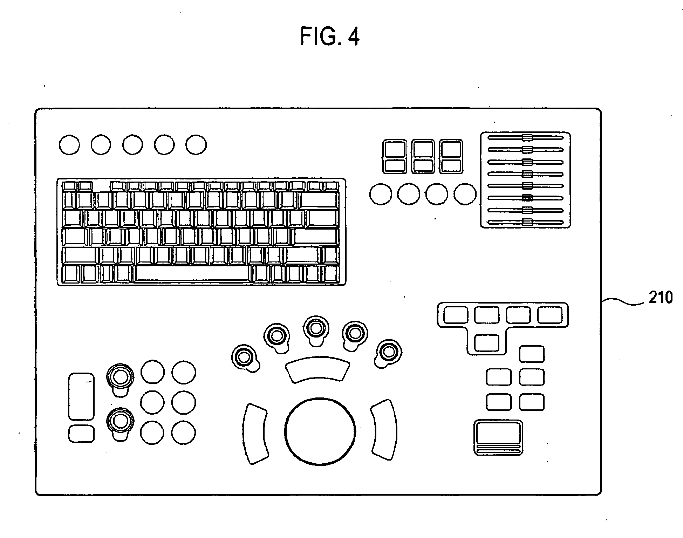

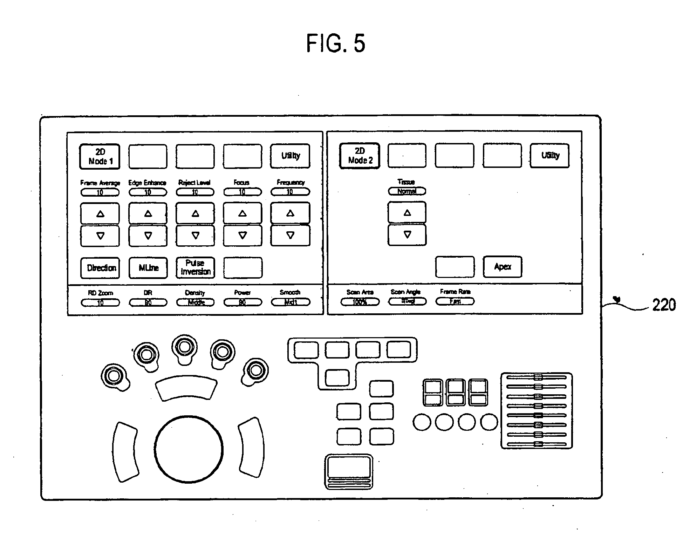

According to one embodiment of the present invention, the touch panel 110 may display a main control panel screen 210 and a corresponding sub control panel screen 220, as shown in FIGS. 4 and 5, respectively. The main control panel screen 210 may provide an initial screen of the portable ultrasound system 100 with a plurality of input sections including: a plurality of buttons for receiving inputs to perform various functions (e.g., to control and operate the portable ultrasound system 100, to set up the display modes of images displayed on the display unit 130 such as B-mode (brightness mode), M-mode (motion mode), C-mode (color mode) and D-mode (Doppler mode), and to adjust the displayed images); a keyboard for receiving text information; a track ball for moving a cursor on the display unit 130 and searching data and images stored in a storage unit; and a handwriting recognition unit for recognizing handwriting inputs on the touch panel 110. For example, the touch panel 110 may display a main control panel screen 210 to allow a user to change the depth, direction and width of two-dimensional images and a sub control panel screen 220 with a plurality of input sections for controlling the optimization of two-dimensional images and enlarging/reducing the images.

The touch panel 110 may recognize external inputs by using the pressure sensing mechanism and/or the electromagnetic induction mechanism.

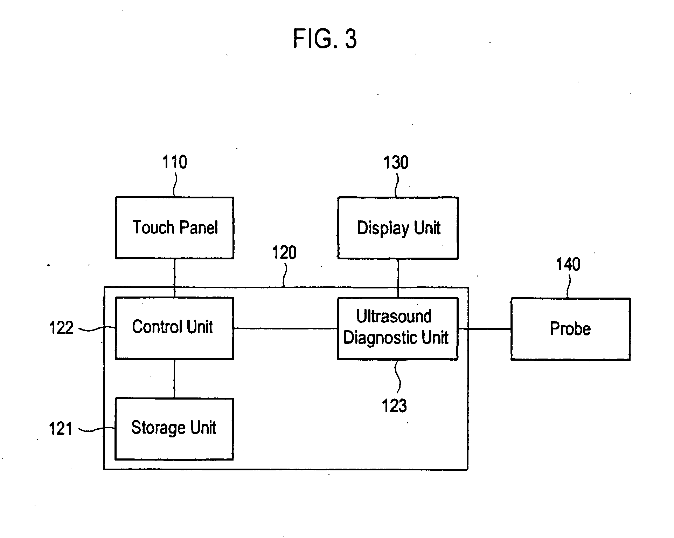

As shown in FIG. 3, the main unit 110 comprises a storage unit 121, a control unit 122 and an ultrasound diagnostic unit 123.

The storage unit 121 stores program codes for generating the main control panel screen 210 and the sub control panel screen 220.

The control unit 122 executes the program codes stored in the storage unit 120 to generate the main control panel screen 210 and the sub control panel screen 220 so that the generated control panel screens are displayed on the touch panel 110. The control unit 122 controls the ultrasound diagnostic unit 123 based on the output signals from the touch panel 110.

The ultrasound diagnostic unit 123 transmit-focuses ultrasound signals transmitted through the probe 140 and receive-focuses the ultrasound signals received through the probe 140 to generate frame data based on the received signals. This is so that the two or three-dimensional images of a target object are formed based on the frame data.

The display unit 130 displays the data and images processed in the ultrasound diagnostic unit 123.

The probe 140 comprises at least one transducer (not shown). The transducers transmit ultrasound signals to the target object and receive the ultrasound signals reflected from the target object.

In accordance with one embodiment of the present invention, there is provided a portable ultrasound system comprising a touch panel for displaying a control panel screen with a plurality of input sections and generating output signals in response to user inputs.

In accordance with one embodiment of the present invention, there is provided a portable ultrasound system, comprising: an ultrasound diagnostic unit for transmitting ultrasound signals to a target object and receiving ultrasound signals reflected from the target object to generate an ultrasound image of the target object based on the received ultrasound signals; a control unit for generating a control panel screen with a plurality of input sections for receiving user inputs to operate said ultrasound diagnostic unit; and a touch panel for displaying the generated control panel screen and generating output signals in response to the user inputs, wherein said control unit is further configured to control said ultrasound diagnostic unit based on the output signals.

In accordance with one embodiment of the present invention, there is provided a portable ultrasound system comprising an ultrasound diagnostic unit, a touch panel and a control unit. The ultrasound diagnostic unit transmits ultrasound signals to a target object and receives the ultrasound signals reflected from the target object to generate an ultrasound image of the target object based on the received ultrasound signals. The touch panel displays a control panel screen with a plurality of input sections to control and operate said ultrasound diagnostic unit and generates output signals from external inputs. The control unit generates the control panel screen and controls said ultrasound diagnostic unit based on the output signals.

Any reference in this specification to "one embodiment," "an embodiment," "example embodiment," etc., means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art. For example, the configuration and arrangement of the input sections included in the control panel screens 210 and 220 may be modified by those skilled in the art.

## Claims

1. A portable ultrasound system (100), comprising: an ultrasound diagnostic unit (123) for transmitting ultrasound signals to a target object and receiving ultrasound signals reflected from the target object to generate an ultrasound image of the target object based on the received ultrasound signals, a control unit (122) and a touch panel (110),
**characterized in that**
the control unit (122) is configured to generate a main control panel screen (210) with a plurality of input sections including a plurality of buttons, a keyboard, a track ball and a handwriting recognition unit for receiving user inputs to operate said ultrasound diagnostic unit, said control unit being configured to further generate a sub control panel screen (220) with a plurality of input sections for controlling the optimization of the ultrasound image and enlarging/reducing the ultrasound image;
the touch panel (110) is configured to display the main control panel screen (210) as an initial screen and generating output signals in response to the user inputs, said touch panel being configured to further display the sub control panel screen (220), wherein said control unit is further configured to control said ultrasound diagnostic unit (123) based on the output signals; and
further comprising a display unit (130) for displaying the ultrasound image.

2. The portable ultrasound system (100) of Claim 1, wherein said touch panel (110) is configured to sense the user inputs by using at least one of a pressure sensing mechanism and an electromagnetic induction mechanism.

3. The portable ultrasound system (100) of Claim 1, further comprising a storage unit (121) for storing program codes to generate said control panel screen.

## Patentansprüche

1. Tragbares Ultraschallsystem (100), welches Folgendes aufweist: eine Ultraschalldiagnoseeinheit (123) zum Übertragen von Ultraschallsignalen auf ein Zielobjekt und zum Empfangen von von dem Zielobjekt reflektierten Ultraschallsignalen, um ein Ultraschallbild des Zielobjekts basierend auf den empfangenen Ultraschallsignalen zu erzeugen, eine Steuereinheit (122) und ein Bildschirm-Tastfeld (110) ,
**dadurch gekennzeichnet, dass**
die Steuereinheit (122) dafür vorgesehen ist, einen Hauptschalttafelbildschirm (210) mit einer Vielzahl von Eingabeabschnitten einschließlich einer Vielzahl von Knöpfen, einer Tastatur, einer Steuerkugel und einer Handschrifterkennungseinheit zum Empfangen von Benutzereingaben, um die Ultraschalldiagnoseeinheit zu bedienen, zu erzeugen wobei die Steuereinheit dafür vorgesehen ist, des weiteren einen Unterschalttafelbildschirm (220) mit einer Vielzahl von Eingabeabschnitten zum Steuern der Optimierung des Ultraschallbilds und Vergrößern bzw. Verkleinern des Ultraschallbilds zu erzeugen;
das Bildschirmtastfeld (110) ist dafür vorgesehen, den Hauptschalttafelbildschirm (210) als ein Einstiegsbild anzuzeigen und als Reaktion auf die Benutzereingaben Ausgabesignale zu erzeugen, wobei das Bildschirmtastfeld dafür vorgesehen ist, des weiteren den Unterschalttafelbildschirm (220) anzuzeigen, wobei die Steuereinheit des weiteren dafür vorgesehen ist, die Ultraschalldiagnoseeinheit (123) basierend auf den Ausgabesignalen zu steuern; und
sie außerdem eine Anzeigeeinheit (130) zum Anzeigen des Ultraschallbilds aufweist.

2. Tragbares Ultraschallsystem (100) nach Anspruch 1, wobei das Bildschirmtastfeld (110) dafür vorgesehen ist, die Benutzereingaben unter Verwendung von wenigstens einem eines Druckerkennungsmechanismus und eines elektromagnetischen Induktionsmechanismus zu erkennen.

3. Tragbares Ultraschallsystem (100) nach Anspruch 1, welches des weiteren eine Speichereinheit (121) zum Speichern von Programmcodes aufweist, um den Schalttafelbildschirm zu erzeugen.

## Revendications

1. Système à ultrasons portatif (100) comprenant une unité de diagnostic à ultrasons (123) servant à transmettre des signaux ultrasonores à un objet cible et à recevoir des signaux ultrasonores réfléchis par l'objet cible pour générer une image ultrasonore de l'objet cible sur la base des signaux ultrasonores reçus, une unité de commande (122) et un panneau tactile (110),
**caractérisé en ce que**
l'unité de commande (122) est configurée pour générer un écran de panneau de contrôle principal (210) comportant une pluralité de sections d'entrée qui comprennent une pluralité de boutons, un clavier, une souris et une unité de reconnaissance de l'écriture manuelle destinée à recevoir des entrées de l'utilisateur pour commander ladite unité de diagnostic ultrasonore, ladite unité de commande étant configurée pour générer en outre un écran de panneau de commande secondaire (220) qui comporte une pluralité de sections d'entrée destinées à commander l'optimisation de l'image ultrasonore et à élargir ou réduire l'image ultrasonore;
le panneau tactile (110) est configuré pour afficher l'écran de panneau de commande principal (210) comme écran initial et pour générer des signaux de sortie en réponse aux entrées de l'utilisateur, ledit panneau tactile étant configuré pour afficher en outre l'écran de panneau de commande secondaire (220), dans lequel ladite unité de commande est en outre configurée pour commander ladite unité de diagnostic ultrasonore (123) sur la base des signaux de sortie ; et
comprenant en outre une unité d'affichage (130) destinée à afficher l'image ultrasonore.

2. Système à ultrasons portatif (100) de la revendication 1, dans lequel ledit panneau tactile (110) est configuré pour détecter les entrées de l'utilisateur en se servant d'au moins l'un d'un mécanisme sensible à la pression et d'un mécanisme à induction électromagnétique.

3. Système à ultrasons portatif (100) de la revendication 1, comprenant en outre une unité de stockage (121) destinée à stocker des codes de programmes pour générer ledit écran de panneau de commande.
